Europäisches Patentamt

European Patent Office

Office européen des brevets

(11) Numéro de publication: **0 342 106**
**A1**

## (12) DEMANDE DE BREVET EUROPEEN

(21) Numéro de dépôt: 89401264.0

(22) Date de dépôt: 03.05.89

(51) Int. Cl.⁴: **A 61 K 31/55**
A 61 K 9/22, A 61 K 9/32

(30) Priorité: 09.05.88 FR 8806238

(43) Date de publication de la demande:
15.11.89 Bulletin 89/46

(84) Etats contractants désignés:
AT BE CH DE ES GB GR IT LI LU NL SE

(71) Demandeur: DELALANDE S.A.
32, rue Henri Regnault
F-92400 Courbevoie (FR)

(72) Inventeur: Cassière, Jean-Pierre
57, Hameau de Bois-Préau
F-92500 Rueil Malmaison (FR)

Ego, Dominique
Résidence West End 280, avenue du Maréchal Juin
F-92100 Boulogne (FR)

Rovei, Vincenzo
44, rue Danton
F-92500 Rueil Malmaison (FR)

(74) Mandataire: Kedinger, Jean-Paul et al
c/o Cabinet Malemont 42, avenue du Président Wilson
F-75116 Paris (FR)

Claims for the following Contracting States:ES + GR

(54) **Comprimés de sel (s) hydrosolubie(s) de Diltiazem à libération programmée et leur procédé de programmation.**

(57) Comprimé pharmaceutique à libération programmée pour l'administration par voie orale d'au moins un sel hydrosoluble de Diltiazem, caractérisé en ce qui'il comprend :

a) une matrice hydrophile représentant 95 à 99,9 % en poids du comprimé et essentiellement constituée :- d'une quantité thérapeutiquement efficace d'au moins un sel hydrosoluble de Diltiazem,
- de 5 à 20 % en poids d'au moins un polymère cellulosique hydrophile choisi dans le groupe comprenant les hydroxyéthylcelluloses,
- de 2 à 5 % en poids de polyvidone,
- de 0,5 à 4 % en poids d'un agent lubrifiant choisi entre le stéarate de magnésium, l'huile de ricin hydrogénée et leurs mélanges, et
- de 20 à 35 % en poids de phosphate dicalcique, et

b) un film entourant ladite matrice, représentant de 0,1 à 5 % en poids du comprimé et essentiellement constitué :
- de 40 à 90 % en poids d'un copolymère neutre ou acide d'acrylate d'éthyle et de méthacrylate de méthyle,
- de 10 à 50 % en poids de polyéthylène glycol 6000, et
- de 0 à 10 % en poids d'hydroxypropylméthylcellulose.

EP 0 342 106 A1

## Description

## Comprimés de sel(s) hydrosoluble(s) de Diltiazem à libération programmée et leur procédé de fabrication

La présente invention a pour objet un comprimé pharmaceutique à libération programmée pour l'administration par voie orale d'au moins un sel hydrosoluble du Diltiazem.

La Diltiazem et notamment ses sels hydrosolubles, en particulier le chlorhydrate de Diltiazem, sont des composés bien connus comme antagonistes calciques utilisés notamment dans le traitement préventif des crises d'angine de poitrine (en particulier l'angor d'effort, l'angor spontané dont l'angor de Prinzmetal), comme antihypertenseurs et comme antiarythmiques.

A ce jour, le chlorhydrate de Diltiazem est commercialisé sous la forme de comprimés à libération immédiate dosés à 60 mg de principe actif et administrés à raison de 3 unités par jour pour être en accord avec la posologie thérapeutique efficace qui est de l'ordre de 180 mg de principe actif/jour.

Il est toutefois souhaitable de disposer de formes pharmaceutiques permettant de réduire, dans toute la mesure du possible, les fluctuations dans le temps des concentrations plasmatiques en principe actif, de manière à conserver une concentration plasmatique efficace sur une période de temps la plus longue possible. Il est par ailleurs fortement souhaitable que les concentrations plasmatiques, tout en étant suffisantes pour assurer une efficacité thérapeutique, ne dépassent pas un seuil au-delà duquel apparaissent des signes de toxicité. Il peut enfin être intéressant de diminuer la fréquence des prises dans le but d'assurer le confort du patient et, partant, de conférer un certain attrait à la spécialité possédant cette caractéristique.

La Demanderesse a donc cherché à atteindre ces buts et pour ce faire, elle a étudié la mise au point d'une spécialité à libération programmée. Elle a ainsi abouti à la réalisation d'une nouvelle forme pharmaceutique permettant une libération régulière et progressive du principe actif, assurant la prolongation de l'activité de ce dernier. Elle a plus précisément abouti à la mise au point d'un comprimé pharmaceutique à libération programmée pour l'administration par voie orale d'au moins un sel hdyrosoluble de Diltiazem, qui est caractérisé en ce qu'il comprend :

a) une matrice hydrophile représentant 95 à 99,9 % en poids du comprimé et essentiellement constituée :
- d'une quantité thérapeutiquement efficace d'au moins un sel hydrosoluble de Diltiazem,
- de 5 à 20 % en poids d'au moins un polymère cellulosique hydrophile choisi dans le groupe comprenant les hydroxyéthylcelluloses,
- de 2 à 5 % en poids de polyvidone,
- de 0,5 à 4 % en poids d'un agent lubrifiant choisi entre le stéarate de magnésium, l'huile de ricin hydrogénée et leurs mélanges, et
- de 20 à 35 % en poids de phosphate dicalcique, et
b) un film entourant ladite matrice, représentant de 0,1 à 5 % en poids du comprimé et essentiellement constitué :
- de 40 à 90 % en poids d'un copolymère neutre ou acide d'acrylate d'éthyle et de méthacrylate de méthyle,
- de 10 à 50 % en poids de polyéthylène glycol 6000, et
- de 0 à 10 % en poids d'hydroxypropylméthylcellulose.

Dans sa conception la plus simple, le comprimé selon l'invention comprend donc une matrice qui contient, outre le principe actif, une matière polymère cellulosique hydrophile, de la polyvidone (jouant le rôle d'agent liant), un agent lubrifiant et du phosphate dicalcique (jouant le rôle de diluant insoluble), et un film en copolymère d'acrylate d'éthyle et de méthacrylate de méthyle, chargé en un polyéthylène glycol spécifique (jouant le rôle de plastifiant et d'agent de perméabilité) et éventuellement en hydroxypropylméthylcellulose (agent de modulation de la perméabilité).

Selon un mode de réalisation préféré de l'invention, le principe actif est constitué par du chlorhydrate de Diltiazem.

Le comprimé selon l'invention pourra contenir de 60 à 250 mg de chlorhydrate de Diltiazem de granulométrie qui permette d'effectuer sans problème la préparation du comprimé. Une formulation particulièrement intéressante est constituée par un comprimé dosé à 120 mg dudit chlorhydrate, qui permet, tout en présentant un faible poids, d'atteindre la posologie thérapeutiquement active.

Par ailleurs, il est préférable selon l'invention, que lorsque, au sein de la matrice, la proportion du polymère cellulosique hydrophile varie de 5 à 20 % en poids ; en d'autres termes, au sein de la matrice, la somme de la proportion du polymère hydroxyéthylcellulosique et de la proportion du phosphate dicalcique est de préférence égale à environ à 40 % en poids.

Le polymère cellulosique hydrophile de la matrice est avantageusement constitué par une hydroxyéthylcellulose de forte viscosité, et notamment une hydroxyéthylcellulose possédant une viscosité Brookfield en solution aqueuse à 1 % (poids/volume) à 25 °C de 3400 à 5000 mPa.s.

L'hydroxypropylméthylcellulose entrant dans la composition du film, présente avantageusement une faible viscosité et en particulier une viscosité Brookfield en solution aqueuse à 2 % (poids/volume) à 20 °C de 2 à 100 mPa.s.

Par ailleurs, on préfère que l'hydroxyéthylcellulose de la matrice possède une granulométrie telle qu'au moins 99,5 % des particules passent à travers un tamis d'une ouverture de maille de 250 μm et que

l'hydroxypropylméthylcellulose du film possède une granulométrie telle qu'au moins 99,5 % des particules passent à travers un tamis d'une ouverture de maille de 420 μm.

A titre d'hydroxyéthylcellulose, on peut citer par exemple, celle commercialisée en France par la société Hercules sous la marque Natrosol HHX et à titre d'hydroxypropylméthylcellulose, on peut citer par exemple, celle commercialisée en France par la société Shinetsu ou Seppic sous la marque Pharmacoat 606.

Le copolymère neutre ou acide d'acrylate d'éthyle et de méthacrylate de méthyle, disponible sur le marché par exemple sous la marque Eudragit L 30D (copolymère acide) ou Eudragit NE 30D (copolymère neutre), peut être utilisé sous la forme d'une dispersion aqueuse.

Quant aux autres constituants (polyvidone, phosphate dicalcique...etc.), ils seront utilisés sous une forme analogue à celle sous laquelle ils sont employés pour la fabrication des comprimés conventionnels.

Enfin, le comprimé selon l'invention peut être de forme plate ou oblongue ou présenter la forme d'un bâtonnet ; en outre, il peut être sécable.

A titre d'exemples spécifiques du comprimé selon l'invention, on peut citer les compositions suivantes :

Matrice :

Composition A :

- Diltiazem                              120 mg
- Hydroxyéthylcellulose          27 mg
- Polyvidone                             5 mg
- Huile de ricin                              2,2 mg
hydrogénée
- Stéarate de                                2,2 mg
magnésium
- Phosphate dicalcique          58,6 mg

Film :

Composition 1 (film représentant 3,2 % en poids du comprimé)

- Copolymère neutre d'acrylate d'éthyle et de méthacrylate de méthyle (Eudragit NE 30D)     6,071 mg

- PEG 6000     0,809 mg

Composition 2 (film représentant 0,5 % en poids du comprimé)

- Copolymère acide d'acrylate

d'éthyle et de méthacrylate

de méthyle (Eudragit L 30D)     0,86 mg

- PEG 6000     0,215 mg

Composition 3 (film représentant 0,66 % en poids du comprimé)

- Copolymère acide d'acrylate

d'éthyle et de methacrylate

de méthyle (Eudragit L 30D)     1,06 mg

- PEG 6000     0,335 mg

- Hydroxypropylmethylcellulose

(28 à 30 % en poids de groupes

méthyle et 7 à 12 % en poids de

groupes hydroxypropyle)     0,015 mg

En ce qui concerne maintenant la préparation du comprimé selon l'invention, il est avantageux de mettre en oeuvre le procédé suivant :

a) Dans un mélangeur de taille convenable, type Lödige, introduire le Diltiazem on un sel hydrosoluble de celui-ci, l'hydroxyéthylcellulose, le phosphate dicalcique et la polyvidone puis mélanger jusqu'à l'obtention d'un mélange homogène,

b) Ajouter au mélange obtenu en a), sous agitation, la quantité d'eau nécessaire à l'obtention d'un granulé,

c) Sécher le granulé obtenu en b), à une température de 55° C environ, afin d'obtenir une humidité résiduelle du granulé inférieure à 2 %,

d) Calibrer le granulé obtenu en c) sur une grille d'ouverture de maille de 800 μm,

e) Introduire dans un mélangeur le granulé obtenu en d), l'huile de ricin hydrogénée et le stéarate de magnésium et agiter jusqu'à homogénéité,

f) Comprimer le mélange obtenu en e), en comprimés de poids unitaire de 215 mg environ, format bâtonnet (L = 13 mm, l = 4,5 mm, r = 3,5 mm),

g) Introduire les comprimés obtenus en f) dans une turbine et pelliculer en continu ces comprimés avec la suspension d'enrobage (mélange d'une dispersion aqueuse du copolymère neutre ou acide d'acrylate d'éthyle et de méthacrylate de méthyle, de PEG 6000, d'eau et éventuellement d'hydroxypropylméthylcellulose) en séchant à température ambiante, pour former le film.

Des études de cinétique de dissolution in vitro ainsi que des études pharmacocinétiques chez l'Homme ont par ailleurs été réalisées sur les comprimés selon l'invention, et notamment dans le cas où le principe actif était constitué par le chlorhydrate de Diltiazem.

En ce qui concerne les études de cinétique de dissolution in vitro, on a fait appel à la méthode II de l'USP XXI (méthode de la palette tournante), les milieux d'épreuve utilisés étant :
- le milieu gastrique artificiel décrit dans l'USP XXI (sans pepsine) ; durée de dissolution : 2 heures ;
- le milieu intestinal artificiel décrit dans l'USP XXI (sans pancréatine) jusqu'au terme de la dissolution.

Les valeurs obtenues, constituées par la moyenne des résultats obtenus sur 6 comprimés, montrent un dissolution du principe actif étalée sur 10 à 12 heures environ, en particulier pour les comprimés dosés à 120 mg de principe actif comportant une matrice ayant la composition A ci-dessus et un film ayant la composition 1 ci-dessus.

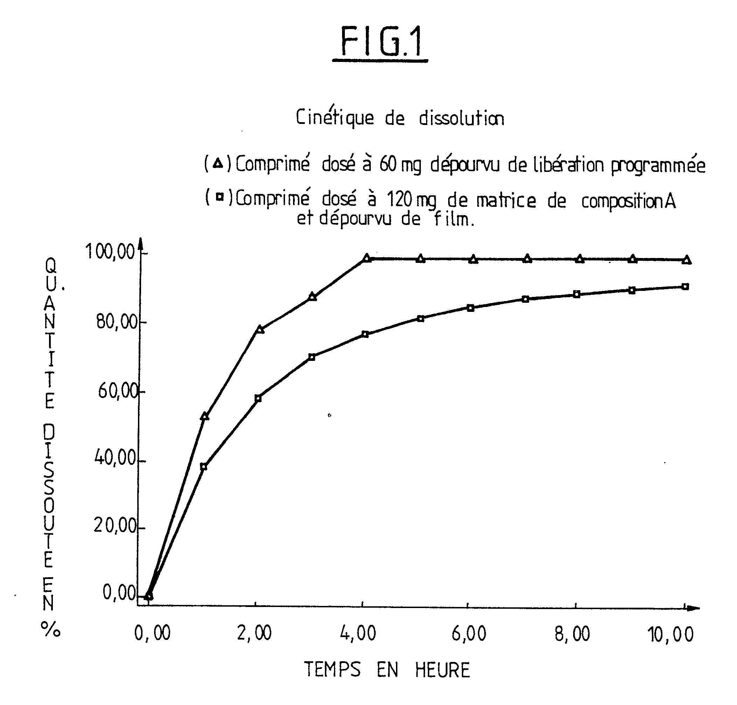

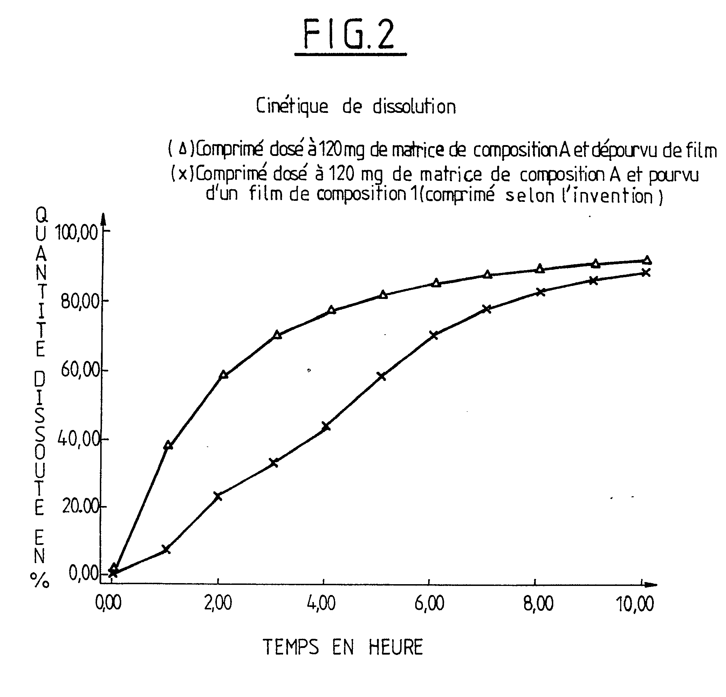

Ceci est clairement mis en évidence par les figures 1 et 2 annexées qui sont constituées par des graphes donnant la quantité (en % en poids) de chlorhydrate de Diltiazem dissous en fonction du temps (heures).

La figure 1 correspond plus précisément à l'étude d'un comprimé de référence dosé à 60 mg et dépourvu de libération programmée et d'un autre comprimé de référence dosé à 120 mg de principe actif de matrice de composition A dépourvu de film.

La figure 2 correspond plus précisément à l'étude des comprimés dosés à 120 mg de principe actif de matrice de composition A dont l'un est dépourvu de film et l'autre est pourvu du film de composition 1.

4

Ces figures 1 et 2 montrent que le film déposé sur le comprimé matriciel permet de réduire considérablement la dissolution du principe actif en milieu gastrique où il est très soluble. Après hydratation, la matrice hydrophile contribue à la libération du principe en milieu intestinal et la libération obtenue est nettement plus progressive (figure 2).

PHARMACOCINETIQUE

Une étude de pharmacocinétique chez 12 adultes volontaires sains a permis de déterminer les paramètres pharmacocinétiques du comprimé selon l'invention.

Les concentrations plasmatiques de Diltiazem ont été déterminées par chromatographie en phase gazeuse.

Ces paramètres ont été comparés à ceux de formulations à libération immédiate (solution aqueuse et gélule) et à ceux d'un comprimé à libération programmée classique, les données relatives auxdites formulations à libération immédiate et audit comprimé à libération programmée classique étant extraites de la littérature.

Les différentes formulations ont été administrées chez le sujet sain, à la même dose de 120 mg.

Le tableau ci-dessous rassemble les résultats des différentes études, tableau dans lequel les symboles Cpmax., Tmax., (1) et (2) ont les significations suivantes :

Tableau

Cpmax. ; concentration plasmatique maximale (moyenne ± écart type),

Tmax. : temps au pic de concentration maximale (moyenne ± écart type),

(1) : OCHS, H.R. et KöLLE, E.U. ; Absolute bioavailability of Diltiazem ; in Fleckenstein et al. (Eds) New Calcium Antagonists ; Recent Developments and Prospects, pp. 225-228 (Gustav Fischer Verlag. Stuggart 1983).

(2) : ROVEI, V. ; GOMENI, R ; MITCHARD, M ; LAURRIBAUD, J ; BLATRIX, Ch. ; THEBAULT, J.J. and MORSELLI, P.L. ; Pharmacokinetics and metabolism of Diltiazem in man. Acta Cardiologica 35, 35-45 (1980).

| Référence bibliographie | Formulation | Cpmax (µg/l) | Tmax (h) |
|---|---|---|---|
| - | Comprimé à libération programmée selon l'invention | 100±44 | 7,8±1,8 |
| (1) | Solution aqueuse | 251±47 | 0,6±0,3 |
| (2) | Gélule | 218±98 | 1,0±0,5 |
| (1) | Comprimés à libération programmée classique | 61±17 | 2,8±0,9 |

Comme cela ressort du tableau ci-dessus, les concentrations de Diltiazem atteignent leur maximum beaucoup plus tard pour le comprimé à libération programmée selon l'invention que pour les autres formulations (Tmax : 7,8 h contre respectivement pour la solution aqueuse, la gélule et le comprimé : 0,6, 1,0 et 2,8 h). Par ailleurs, les concentrations maximales de Diltiazem sont supérieures à celles observée avec le comprimé à libération programmée classique.

En outre, il a pu être mis en évidence que le profil pharmacocinétique du comprimé selon l'invention fait apparaître un plateau de concentrations plasmatiques survenant en moyenne 5 à 10 heures après l'administration.

On notera également que les concentrations plasmatiques maximales obtenues après administration des formulations à libération immédiate (solution aqueuse et gélule) sont très élevées et dépassent le seuil toxique, l'intervalle thérapeutique étant en effet de 70 à 200 µm/l pour le Diltiazem (voir référence bibliographique (2) ci-dessus).

Tous ces résultats montrent que les concentrations plasmatiques obtenues après administration du comprimé selon l'invention, dosé à 120 mg, sont suffisantes pour assurer une efficacité sur 24 heures (avec une posologie d'un comprimé toutes les douze heures) tout en étant suffisamment faibles pour éviter l'apparition d'éventuels effets toxiques.

## Revendications

1. Comprimé pharmaceutique à libération programmée pour l'administration par voie orale d'au moins un sel hydrosoluble de Diltiazem, caractérisé en ce qu'il comprend :
   a) une matrice hydrophile représentant 95 à 99,9 % en poids du comprimé et essentiellement constituée :
   - d'une quantité thérapeutiquement efficace d'au moins un sel hydrosoluble de Diltiazem,
   - de 5 à 20 % en poids d'au moins un polymère cellulosique hydrophile choisi dans le groupe comprenant les hydroxyéthylcelluloses,
   - de 2 à 5 % en poids de polyvidone,
   - de 0,5 à 4 % en poids d'un agent lubrifiant choisi entre le stéarate de magnésium, l'huile de ricin hydrogénée et leurs mélanges, et
   - de 20 à 35 % en poids de phosphate dicalcique, et
   b) un film entourant ladite matrice, représentant de 0,1 à 5 % en poids du comprimé et essentiellement constitué :
   - de 40 à 90 % en poids d'un copolymère neutre ou acide d'acrylate d'éthyle et de méthacrylate de méthyle,
   - de 10 à 50 % en poids de polyéthylène glycol 6000, et
   - de 0 à 10 % en poids d'hydroxypropylméthylcellulose.

2. Comprimé selon la revendication 1, caractérisé en ce qu'au sein de la matrice, la somme de la proportion du polymère hydroxyéthylcellulosique et de la proportion du phosphate dicalcique est sensiblement égale à 40 % en poids.

3. Comprimé selon la revendication 1 ou 2, caractérisé en ce que le sel hydrosoluble de Diltiazem est le chlorhydrate de Diltiazem.

4. Comprimé selon la revendication 1, 2 ou 3, caractérisé en ce qu'il contient 60 à 250 mg de chlorhydrate de Diltiazem.

5. Comprimé selon la revendication 4, caractérisé en ce qu'il contient 120 mg de chlorhydrate de Diltiazem

6. Comprimé selon l'une des revendications précédentes, caractérisé en ce que le polymère cellulosique hydrophile de la matrice est constitué par une hydroxyéthylcellulose possédant une viscosité Brookfield en solution aqueuse à 1 % (poids/volume) à 25 °C de 3400 à 5000 mPa.s.

7. Comprimé selon l'une des revendications précédentes, caractérisé en ce que l'hydroxypropylméthylcellulose du film possède une viscosité Brookfield en solution aqueuse à 2 % (poids/volume) à 20 °C de 2 à 100 mPa.s.

8. Comprimé selon la revendication 6 ou 7, caractérisé en ce que l'hydroxyéthylcellulose possède une granulométrie telle qu'au moins 99,5 % des particules passent à travers un tamis d'une ouverture de maille de 250 µm et en ce que l'hydroxypropylméthylcellulose possède une granulométrie telle qu'au moins 99,5 % des particules passent à travers un tamis d'une ouverture de maille de 420 µm.

9. Comprimé selon l'une des revendications précédentes, caractérisé en ce que la matrice possède la composition suivante :

| - Diltiazem | 120 mg |
| - Hydroxyéthylcellulose | 27 mg |
| - Phosphate dicalcique | 58,6 mg |
| - Polyvidone | 5 mg |
| - Huile de ricin hydrogénée | 2,2 mg |
| - Stéarate de magnésium | 2,2 mg |

10. Comprimé selon la revendication 9, caractérisé en ce que le film possède une composition choisie parmi les suivantes :

Composition 1 (film représentant 3,2 % en poids du comprimé)

   - Copolymère neutre d'acrylate d'éthyle et de méthacrylate de méthyle (Eudragit NE 30D)                 6,071 mg

   - PEG 6000                                   0,809 mg

Composition 2 (film représentant 0,5 % en poids du comprimé)

   - Copolymère acide d'acrylate

     d'éthyle et de méthacrylate

     de méthyle (Eudragit L 30D)                  0,86 mg

   - PEG 6000                                   0,215 mg

Composition 3 (film représentant 0,66 % en poids du comprimé)

   - Copolymère acide d'acrylate

     d'éthyle et de méthacrylate

     de méthyle (Eudragit L 30D)                  1,06 mg

   - PEG 6000                                   0,335 mg

   - Hydroxypropylméthylcellulose

     (28 à 30 % en poids de groupes

     méthyle et 7 à 12 % en poids de

     groupes hydroxypropyle)                    0,015 mg

**Revendications pour les Etats contractants suivants: AT, ES, GR**

1. Procédé de fabrication d'un comprimé pharmaceutique à libération programmée pour l'administration par voie orale d'au moins un sel hdyrosoluble de Diltiazem, caractérisé en ce qu'il comprend :
    a) la préparation d'une matrice hydrophile représentant 95 à 99,9 % en poids du comprimé et essentiellement constituée :- d'une quantité thérapeutiquement efficace d'au moins un sel hydrosoluble de Diltiazem,
    - de 5 à 20 % en poids d'au moins un polymère cellulosique hydrophile choisi dans le groupe comprenant les hydroxyéthylcelluloses,
    - de 2 à 5 % en poids de polyvidone,
    - de 0,5 à 4 % en poids d'un agent lubrifiant choisi entre le stéarate de magnésium, l'huile de ricin hydrogénée et leurs mélanges, et
    - de 20 à 35 % en poids de phosphate dicalcique, et
    b) le dépôt sur ladite matrice, d'un film représentant de 0,1 à 5 % en poids du comprimé et essentiellement constitué :
    - de 40 à 90 % en poids d'un copolymère neutre ou acide d'acrylate d'éthyle et de méthacrylate de méthyle,
    - de 10 à 50 % en poids de polyéthylène glycol 6000, et
    - de 0 à 10 % en poids d'hydroxypropylméthylcellulose.

2. Procédé selon la revendication 1, caractérisé en ce que la préparation de la matrice comprend :
- le mélange jusqu'à homogénéité, du Diltiazem ou du sel hydrosoluble de celui-ci, de l'hydroxyéthylcellulose, du phosphate dicalcique et de la polyvidone,
- l'adjonction au mélange homogène obtenu, sous agitation, de la quantité d'eau nécessaire à l'obtention d'un granulé,
- le séchage du granulé obtenu, à une température de 55° C environ, afin d'obtenir une humidité

7

EP 0 342 106 A1

résiduelle du granulé inférieure à 2 %,
- le calibrage du granulé séché obtenu, sur une grille d'ouverture de maille de 800 μm,
- l'agitation jusqu'à homogénéité, du granulé calibré en présence de l'huile de ricin hydrogénée et du stéarate de magnésium, puis
- la compression du mélange résultant pour obtenir la matrice.

3. Procédé selon la revendication 2, caractérisé en ce que le dépôt du film sur la matrice comprend le pelliculage en continu de la matrice avec une suspension d'enrobage comprenant un mélange d'une dispersion aqueuse du copolymère neutre ou acide d'acrylate d'éthyle et de méthacrylate de méthyle, de PEG 6000, d'eau et éventuellement d'hydroxypropylméthylcellulose, suivi d'un séchage.

4. Procédé selon l'une des revendications 1 à 3, caractérisé en ce qu'au sein de la matrice, la somme de la proportion du polymère hydroxyéthyloellulosique et de la proportion du phosphate dicalcique est sensiblement égale à 40 % en poids.

5. Procédé selon l'une des revendications 1 à 4, caractérisé en ce que le sel hydrosoluble de Diltiazem est le chlorhydrate de Diltiazem.

6. Procédé selon l'une des revendications précédentes, caractérisé en ce que le polymère cellulosique hydrophile de la matrice est constitué par une hydroxyéthylcellulose possédant une viscosité Brookfield en solution aqueuse à 1 % (poids/volume) à 25 °C de 3400 à 5000 mPa.s.

7. Procédé selon l'une des revendications précédentes, caractérisé en ce que l'hydroxypropylméthylcellulose du film possède une viscosité Brookfield en solution aqueuse à 2 % (poids/volume) à 20 °C de 2 à 100 mPa.s.

8. Procédé selon la revendication 6 ou 7, caractérisé en ce que l'hydroxyéthylcellulose possède une granulométrie telle qu'au moins 99,5 % des particules passent à travers un tamis d'une ouverture de maille de 250 μm et en ce que l'hydroxypropylméthylcellulose possède une granulométrie telle qu'au moins 99,5 % des particules passent à travers un tamis d'une ouverture de maille de 420 μm.

9. Procédé selon l'une des revendications précédentes, caractérisé en ce que la matrice possède la composition suivante :

| | |
|---|---|
| - Diltiazem | 120 mg |
| - Hydroxyéthylcellulose | 27 mg |
| - Phosphate dicalcique | 58,6 mg |
| - Polyvidone | 5 mg |
| - Huile de ricin hydrogénée | 2,2 mg |
| - Stéarate de magnésium | 2,2 mg |

10. Procédé selon la revendication 9, caractérisé en ce que le film possède une composition choisie parmi les suivantes :

Composition 1 (film représentant 3,2 % en poids du comprimé)

| | |
|---|---|
| - Copolymère neutre d'acrylate d'éthyle et de méthacrylate de méthyle (Eudragit NE 30D) | 6,071 mg |

8

  - PEG 6000                                              0,809 mg

<u>Composition 2</u> (film représentant 0,5 % en poids du comprimé)

  - Copolymère acide d'acrylate

    d'éthyle et de méthacrylate

    de méthyle (Eudragit L 30D)                  0,86 mg

  - PEG 6000                                              0,215 mg

<u>Composition 3</u> (film représentant 0,66 % en poids du comprimé)

  - Copolymère acide d'acrylate

    d'ethyle et de méthacrylate

    de méthyle (Eudragit L 30D)                  1,06 mg

  - PEG 6000                                              0,335 mg

  - Hydroxypropylméthylcellulose

    (28 à 30 % en poids de groupes

    méthyle et 7 à 12 % en poids de

    groupes hydroxypropyle)                     0,015 mg

# FIG.1

Cinétique de dissolution

(▲) Comprimé dosé à 60 mg dépourvu de libération programmée

(□) Comprimé dosé à 120 mg de matrice de composition A
et dépourvu de film.

EP 0 342 106 A1

# FIG.2

Cinétique de dissolution

( Δ )Comprimé dosé à 120 mg de matrice de composition A et dépourvu de film
(x)Comprimé dosé à 120 mg de matrice de composition A et pourvu
d'un film de composition 1(comprimé selon l'invention )

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | EP-A-0 248 447   (WARNER LAMBERT) <br> * exemples 1-7;  revendications * <br> --- | 1-10 | A 61 K   31/55 <br> A 61 K    9/22 <br> A 61 K    9/32 |
| Y | EP-A-0 068 446   (GOEDECKE) <br> * page 7, exemple 2 * <br> --- | 1-10 | |
| Y | EP-A-0 207 638   (TEJIN) <br> * pages 3,5 * <br> --- | 1-10 | |
| Y | EP-A-0 214 735   (EUROCELTIQUE) <br> * revendications 3-6 * <br> --- | 1-10 | |
| Y | PATENT ABSTRACTS OF JAPAN <br> vol. 11, no. 184 (C-427)(2631), 12 juin <br> 1987; & JP - A - 62 5915 (NIPPON <br> IYAKUHIN KOGYO) 12.01.1987 <br> --- | 1-10 | |
| Y | EP-A-0 077 956   (TANABE SEIYAKU) <br> * page 11, revendications * <br> --- | 1-10 | |
| A | EP-A-0 247 634   (WARNER LAMBERT) <br> * revendications * <br> ----- | 1-10 | DOMAINES TECHNIQUES RECHERCHES (Int. Cl.4) <br><br> A 61 K    9/00 <br> A 61 K   31/00 |

Le présent rapport a été établi pour toutes les revendications

| Lieu de la recherche | Date d'achèvement de la recherche | Examinateur |
|---|---|---|
| BERLIN | 24-07-1989 | AVEDIKIAN P.F. |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons
........................................................................
& : membre de la même famille, document correspondant

EPO FORM 1503 03.82 (P0402)